# FASCICULE DE BREVET EUROPEEN

(11) **EP 0 840 624 B1**
(45) Date de publication et mention de la délivrance du brevet: **25.07.2007**
(21) Numéro de dépôt: 96923791.6
(22) Date de dépôt: 15.07.1996
(51) Int. Cl.: A61L 2/10, A61K 35/14, A61L 2/24

(54) **INSTALLATION D'INACTIVATION DE CONTAMINANTS VIRAUX PRESENTS DANS DES PRODUITS SANGUINS**
VORRICHTUNG ZUR INAKTIVIERUNG VON VIRALEN KONTAMINATIONEN IN BLUTPRODUKTEN
APPARATUS FOR INACTIVATING VIRAL CONTAMINANTS IN BLOOD PRODUCTS

(30) Priorité: 14.07.1995 WO PCT/BE95/00069
(43) Date de publication de la demande: 13.05.1998
(73) Titulaire: CAF - DCF cvba - scrl, 1120 Bruxelles (BE)
(72) Inventeur: LAUB, Ruth, B-1190 Bruxelles (BE); DE WAEL, Luc, B-2520 Ranst (BE); DI GIAMBATTISTA, Mario, B-7090 Braine-le-Comte (BE)
(74) Mandataire: De Clercq, Ann G. Y.
(86) Numéro de dépôt international: PCT/BE1996/000076
(87) Numéro de publication internationale: WO 1997/003706

(56) Documents cités:
- EP-A2- 0 018 561
- EP-A2- 0 311 950
- WO-A-00/20045
- WO-A1-94/03054
- WO-A1-94/28120
- WO-A1-95/00631
- WO-A1-96/02571
- GB-B- 2 200 020
- VOX SANG. (1993), 64(2), 82-8, 1993, XP000608397 HART, H. ET AL: "Inactivation of viruses during ultraviolet light treatment of human intravenous immunoglobulin and albumin"
- PHOTOCHEM. PHOTOBIOL. (1996), 63(4), 541-6, 1996, XP000607648 MARX, GERARD ET AL: "Protecting fibrinogen with rutin during UVC irradiation for viral inactivation"
- BLOOD 86 (11). 1995. 4331-4336, XP000608313 CHIN S ET AL: "Virucidal short wavelength ultraviolet light treatment of plasma and factor VIII concentrate: Protection of proteins by antioxidants." cité dans la demande
- LI Q; MACDONALD S; BIENEK C; FOSTER P R; MACLEOD A J: "Design of a UV-C irradiation process for the inactivation of viruses in protein solutions" BIOLOGICALS, ACADEMIC PRESS LTD., vol. 33, no. 2, juin 2005 (2005-06), pages 101-110, XP004918420 LONDON, GB
- CAILLET-FAUQUET ET AL: "Continuous-flow UVC irradiation: a new, effective, protein activity-preserving system for inactivating bacteria and viruses, including erythrovirus B19" JOURNAL OF VIROLOGICAL METHODS, vol. 118, no. 2, 15 juin 2004 (2004-06-15), pages 131-139,
- SCHMIDT S: "Letter to the editor" BIOLOGICALS, vol. 34, no. 3, septembre 2006 (2006-09), pages 237-238, XP005598590 London, GB

## Description

### Objet de l'invention.

La présente invention concerne une installation d'inactivation physique de contaminants viraux présents dans des produits sanguins, notamment le sang total, le plasma, des liquides comprenant des composés cellulaires du sang, et les dérivés sanguins tels que les facteurs de coagulation (facteur VIII, facteur IX, facteur de von Willebrand, ...), le fibrinogène, la fibronectine, les immunoglobulines, l'albumine, ..., y compris les produits non naturels obtenus par génie biologique, ladite installation comprenant les caractéristiques techniques indiquées dans les revendications.

### Arrière-plan technologique à la base de l'invention

La mise à disposition de produits sanguins, nécessite pour leur utilisation à des fins thérapeutiques ou non thérapeutiques, des techniques de purification permettant d'obtenir des produits ce haute pureté, et de préférence dépourvus de contaminants, en particulier de contaminants viraux.

Dans des produits sanguins, les contaminants viraux peuvent être des virus enveloppés (HIV, virus de l'hépatite B, C, D, E, G, ...) ou non enveloppés (virus de l'hépatite A, parvovirus, ...).

Depuis de nombreuses années, différentes instances internationales ou nationales ont instauré des normes de plus en plus sévères pour la préparation des produits sanguins de manière à empêcher leur application à des fins thérapeutiques ou non thérapeutiques, lorsque ceux-ci présentent des contaminants viraux (Directives du Conseil 65/65 EEC, 75/319/EEC & 89/381 EEC).

Au niveau européen, les normes CPMP (CPMP/BWT 268/95 et 269/95) requièrent l'usage de certains traitements à l'encontre des virus enveloppés ou non enveloppés.

Il est notamment mentionné dans ces documents qu'une étape d'inactivation par la chaleur (sèche ou vapeur) ou par pasteurisation dans la préparation des facteurs de coagulation est efficace à l'encontre du virus de l'hépatite A, mais serait peu efficace à l'encontre d'autres virus non enveloppés en particulier les parvovirus.

Par contre, un traitement comportant une étape d'inactivation chimique (par addition de solvants-détergents) est efficace pour les virus enveloppés mais inefficace pour traiter les virus non enveloppés.

Il est également connu d'utiliser certains agents chimiques tels que la bêta-propiolactone qui est efficace dans le traitement des virus non enveloppés, mais présente l'inconvénient de modifier les protéines traitées.

Il est également connu que certains longs traitements par modification du pH, en dessous du pH 4, ou l'addition de protéases permet une activation de certains virus non enveloppés tels que les parvovirus. Cependant, ces traitements modifient également la conformation et la structure des protéines traitées.

En conséquence, il est connu que, à ce jour, la majorité des étapes de traitement physico-chimiques susceptibles d'être utilisés pour obtenir une inactivation virale de produits sanguins sont soit hautement toxiques, soit affectent d'une manière inacceptable la conformation des protéines traitées, soit sont inefficaces pour traiter des virus non enveloppés en particulier les parvovirus.

Les parvovirus sont des petits virus non enveloppés, à DNA qui infectent de nombreuses espèces animales, y compris l'homme (Handbook of Parvoviruses, Vol. 1, pp. 1-30, Desinfection, Sterilization and Preservation, Fourth Edition, Seymour S. Block, Ed. Lea & Febiger, Philadelphia-London). Ils sont endémiques dans la nature et causent une large variété de maladies dont l'apparition dépend largement de l'état de développement de l'hôte.

Parmi ceux-ci, le parvovirus B19 est le seul membre connu de la famille des parvoviridae qui soit pathogène pour l'homme. De même, le parvovirus murin H1 peut également contaminer l'homme.

L'infection par les parvovirus B19 chez l'homme sain peut être asymptomatique ou induire des maladies bénignes (exemple : cinquième maladie chez l'enfant).

Par contre, chez des patients immunodéficients ou atteints de désordres sanguins, il peut conduire à des anémies chroniques et à des aplasies transitoires pouvant être associées à des anémies hémolytiques.

Passant au travers du placenta, il peut provoquer la mort intra-utérine. Il présente un remarquable tropisme pour les lignées érythroïdes de cellules progénitrices hématopoïétique humaine.

Une enquête épidémiologique récente a montré que 50 à 60% de la population adulte française et 36% des enfants de 1 à 15 ans ont une sérologie parvovirus positive.

La présence de DNA viral a été mise en évidence par amplification génétique (PCR) dans un certain nombre de lots de concentrés de facteur VIII purifiés, quels que soient les procédés d'inactivation virale utilisés.

Ceci a été confirmé par la détection de sérologie B19⁺, sans signe clinique, chez 85% des enfant hémophiles n'ayant reçu, depuis leur naissance, que du concentré de facteur VIII hautement purifié (FVIII THPSD) utilisé en France depuis 1988, et exempt de toute contamination par des virus enveloppés (HIV, HBV, HCV) (Y. Lauriau et Al., 1er congrès de la Société Française de Transfusion (1994)).

Cette observation démontre bien que, sans de nouvelles méthodes d'inactivation virale, ciblées sur l'élimination sélective des parvovirus en particulier du parvovirus B19 des produits sanguins, la probabilité de contamination est élevée.

Les parvovirus sont extrêmement résistants, même à haute température. Leurs propriétés d'hémagglutination et leur infectivité ne sont pas affectées par des traitements chimiques tels que le chloroforme, différents acides, et la plupart résistent à des digestions enzymatiques par RNase, DNase, papaïne, trypsine.

### Etat de la technique

La demande internationale de brevet WO95/00631 décrit un procédé d'inactivation virale de produits sanguins comprenant l'addition à ces produits sanguins de produits photoactivables par un rayonnement UVA et qui deviendraient toxiques pour les virus présents dans ces produits sanguins. Ce procédé comportant une étape permettant d'isoler ces réactifs toxiques des produits sanguins de manière à ce que ceux-ci ne soient pas contaminés par ces agents toxiques.

Parmi ces agents toxiques, on mentionne notamment le psoralène.

Cependant, ce procédé présente l'inconvénient que l'on ne peut garantir que les produits sanguins traités ne seront pas complètement dépourvus de ces agents photoactivables qui seraient susceptibles de dénaturer et/ou inactiver les produits sanguins traités et provoquer une toxicité chez l'homme ou l'animal lorsqu'ils sont réinjectés de manière répétitive, même à faible dose, avec les produits sanguins traités.

Il est également connu qu'il est possible de stériliser un grand nombre de produits en les soumettant à un rayonnement ultraviolet. Il est en particulier connu par le document "Sterilization By Ultraviolet Irradiation" (chapter 31, IL SHECHMEISTER) que le rayonnement ultraviolet est susceptible de détruire des contaminants tels que des virus, des mycoplasmes, des bactéries et des champignons. Un tel rayonnement, peut être notamment utilisé dans des milieux tels que des gaz ou des liquides.

Le document WO94/03054 décrit un procédé d'inactivation de microorganismes présents dans les produits sanguins, ledit procédé comprenant l'addition à ces produits de 8-methoxy-psoralène, suivie d'une irradiation des produits aux rayons UV. (180 nm-400 nm) afin de rendre le 8-méthoxypsoralène actif.

Le document WO94/28120 décrit un procédé d'inactivation virale de produits sanguins, comprenant l'irradiation de ces produits aux rayons UVC en présence de composés quencheurs.

Le document de Hart H. et al (Vox Sang 1993; 64: 82-88) décrit l'irradiation par rayons UVC de préparations à base d'albumine et d'immunoglobulines, contaminées par différents types de virus y compris des virus non-enveloppés. L'appareillage utilisé consiste en un émetteur UVC, une bouteille contenant le produit en question ainsi que des dosimètres. La bouteille est soumise à un mouvement rotationnel continu.

Il est également connu par le document de Chin S. et al, (Blood, volume 86, n°11, décembre 1995, p.4331-4336) et par le document de Marx G. et al (Photochemistry and Photobiology, 1996, 63 (11) : 541-546) de traiter des produits sanguins par rayonnement ultraviolet de type C en présence ou en absence d'agents anti-oxydants tels que la rutine et d'obtenir l'inactivation de virus non enveloppés, notamment des parvovirus.

En outre, les procédés d'inactivité virale de l'état de la technique peuvent affecter l'intégrité et l'activité des produits sanguins (en particulier la conformation tridimensionnelle des facteurs de coagulation tels que le facteur VIII) et par conséquent leurs activités.

De plus, les procédés de l'inactivation virale de l'état de la technique présentent souvent des difficultés quant à leur validation, car ils présentent des problèmes de reproductibilité ou de monitorage. En effet, certains paramètres de traitement doivent être modifiés ou ne peuvent aisément être maintenus, en particulier lorsque l'on doit surveiller le degré d'humidité si on effectue un traitement à chaleur sèche. De plus, il est difficile de contrôler les différentes étapes des processus opératoires.

### Buts de l'invention

La présente invention vise à obtenir une installation d'inactivation de contaminants viraux présents dans des produits sanguins, qui ne présente pas les inconvénients de l'état de la technique et qui soit simple, rapide, peu coûteuse et reproductible.

Le procédé d'inactivation virale respecte l'intégrité des produits sanguins, en particulier celle des facteurs de coagulation tels que le facteur VIII, le facteur IX, le facteur de von Willebrand, la fibronectine, le fibrinogène,...

Un but complémentaire de la présente invention vise à obtenir une installation qui soit aisément validable et qui soit conforme aux bonnes pratiques de production pharmaceutique (GMP) et aux normes européennes (CPMP).

Un dernier but de la présente invention vise à obtenir une installation d'inactivation virale de produits sanguins permettant d'inactiver des virus non enveloppés, de préférence à simples brins, tels que les parvovirus, en particulier les parvovirus B19 et H1.

La présente invention concerne une installation pour la préparation d'un produit sanguin comprenant un dispositif assurant une inactivation virale physique, le dispositif comportant :
un un émetteur de rayons ultraviolets de type C (UVC),
   - un tube en quartz ou en un matériau polymérisé ne permettant pas l'absorption des rayonnements ultraviolets de type C, permettant, par un système de turbulence, de maintenir un flux homogène du produit sanguin circulant dans ce tube, dont le diamètre est adapté au volume à traiter afin que les produits sanguins ne soient pas en fines couches par éviter les phénomènes de perturbations au niveau de la surface solide/liquide de ce tube,
   - une enceinte réflectante renvoyant les rayonnements ultraviolets de type C vers le produit sanguin à traiter.

La présente invention concerne aussi cette installation, caractérisée en ce que le dispositif comporte un système de contrôle de la dose d'ultraviolets C qui irradie le produit sanguin à traiter.

La présente invention concerne aussi cette installation, caractérisée en ce que le dispositif comporte un système de contrôle de la température du produit sanguin à traiter.

La présente invention concerne cette installation, caractérisée en ce que la température est contrôlée et enregistrée tant a l'intérieur du dispositif que dans le produit sanguin à traiter.

La présente invention concerne cette installation, caractérisée en ce que les rayons ultraviolets de type C ont une longueur d'ondes comprise entre 250 et 270 nm, de préférence une longueur d'onde de 254 nm.

La présente invention concerne cette installation, caractérisée en ce que l'installation est construite d'un seul tenant ou en modules mobiles juxtaposés placés en série.

La présente invention concerne aussi l'utilisation de cette installation, pour l'inactivation virale de virus non enveloppés présents dans un produit sanguin.

On entend par "produit sanguin", tout produit sanguin, liquide ou solide obtenu de manière naturelle à partir du corps humain ou animal ou par voie de synthèse tel que le sang total, ses composés cellulaires, ses dérivés tels que le sérum ou le plasma et les composés protéiques du sang, à savoir les facteurs de coagulation (facteur VIII, facteur IX, facteur de von Willebrand, ...), le fibrinogène, la fibronectine, les immunoglobulines, l'albumine, ..., y compris des composés protéiques obtenus par génie biologique, tels que des protéines recombinantes ou des peptides de synthèse.

Ces produits peuvent être également des facteurs produits par certaines lignées cellulaires sanguines spécifiques telles que des interférons, des interleukines, ou des récepteurs cellulaires de ces molécules obtenus de manière naturelle ou par voie synthétique notamment les peptides ou protéines recombinantes obtenues par la technique du DNA recombinant. De manière avantageuse, le procédé d'inactivation provoque une inactivation d'agents contaminants tels que des virus non enveloppés (HAV), enveloppés (HIV, virus de l'hépatite B, C, D, E, G, ...), des agents bactériens, ..., éventuellement présents dans le produit sanguin.

Le procédé peut être également combiné à un ou plusieurs traitement(s) additionnel(s) d'inactivation de contaminants notamment viraux bien connu de l'homme de l'art, en particulier les traitements physique ou chimique d'inactivation virale choisis parmi le groupe constitué par une ou plusieurs étape(s) de chauffage sec ou humide, l'addition de composants chimiques, en particulier du solvant-détergent ou des produits devenant actifs sous un rayonnement ultraviolet, une ou plusieurs étape(s) de pasteurisation, la soumission à une ou plusieurs émissions de rayonnement particuliers tels que des rayonnements y ou des rayons X ou une combinaison de ces procédés. Parmi les produits actifs susceptibles d'être additionnés aux produits sanguins, on peut mentionner notamment les agents protecteurs vis-à-vis des radicaux libres (vitamine C, ...) et la beta-propiolactone qui provoque un phénomène d'alkylation des protéines. De tels produits devront être utilisés à des doses ne provoquant pas de phénomène de toxicité ou de dénaturation des produits sanguins traités. Cependant, aux doses d'irradiation utilisées selon l'invention, l'addition de tels produits n'est pas nécessaire pour provoquer une inactivation des virus non enveloppés ou assurer une protection vis-à-vis des radicaux libres.

Le procédé d'inactivation virale peut être combiné à un procédé général d'isolation ou de séparation de dérivés sanguins à partir du sang total.

Ce procédé peut comprendre une ou plusieurs étape(s) de filtration, de précipitation, de séparation par chromatographie, ... permettant de séparer les différents composants du sang total les uns des autres.

Selon l'invention, la majorité de l'émission du rayonnement UVC s'effectue entre 250 et 270 nm, de préférence à la longueur d'onde de 254 nm, c'est-à-dire la zone d'absorption privilégiée des acides nucléiques et les doses d'irradiation reçues par les produits sont comprises entre 10 et 2.000 joules/m², de préférence entre 230 et 400 joules/m².

Dans le procédé on choisit les doses d'irradiation et la longueur d'onde utilisée de manière à ce que les doses d'irradiation reçues par le produit sanguin traité affectent essentiellement les acides nucléiques des contaminants, sans perturber la structure des peptides ou des protéines présentes dans le produit sanguin traité.

De manière inattendue, les Inventeurs ont observé qu'il était possible de traiter des produits sanguins en fines couches ("monolayers" ou couches dites laminaires) ou non, c'est-à-dire qu'il n'existe pas de facteurs limitatifs des volumes traités. Cette propriété est particulièrement avantageuse car en traitant des produits sanguins qui ne sont pas en fines couches, il est possible d'éviter les phénomènes de perturbation existant au niveau de la surface solide/liquide lorsque l'on travaille en fines couches. En outre, en ne travaillant pas en fines couches, il est possible de traiter de grandes quantités de produits sanguins et d'éviter les problèmes d'échauffement et de cisaillement des produits traités (BAILEY, Bioch.Fond ,McGraw-Hill)

La longueur d'onde de l'émission de rayonnement UVC et les doses d'irradiation peuvent être adaptées par l'homme du métier en fonction de la quantité et du type de produits sanguins à traiter. Il est à noter que plus les doses d'irradiation reçues par le produit sanguin à traiter seront élevées, plus l'inactivation des contaminants présents sera assurée. Cependant, de manière à réduire le phénomène de dénaturation du produit sanguin, l'homme du métier adaptera la dose d'irradiation de la longueur d'onde de l'émission UVC de manière à réduire la dénaturation et la perte d'activité desdits produits sanguins. Cette adaptation se fera de manière à être conforme aux normes européennes CPMP (CPMP/BWP268/95 et 269/95).

Il est possible d'obtenir une inactivation virale totale des parvovirus présents (c'est-à-dire que l'on ne peut plus identifier de virus au-dessus du seuil de détection) en limitant les doses d'irradiation reçues et en permettant une réduction de perte d'activité dudit produit inférieure à 10-15%, de préférence inférieure à 5%.

L'installation de l'invention comporte également des dispositifs assurant l'isolation ou la séparation de dérivés sanguins à partir du gang total.

Ces dispositifs pouvant comprendre des moyens de précipitation, de centrifugation/décantation, de filtration, de concentration, de dialyse du produit sanguin à traiter et adaptables par l'homme du métier en fonction des produits sanguins séparés et traités.

De préférence, le produit sanguin mis en contact avec le rayonnement ultraviolet C, est disposé dans un tube en quartz ou en un matériau polymérisé et généralement non absorbant dans la zone de longueur d'onde émise par les rayonnements ultraviolets C. L'installation peut également comprendre un dispositif permettant l'addition dans le produit sanguin, d'un agent protecteur vis-à-vis des radicaux libres susceptibles d'être générés par le rayonnement ultraviolet. De tels agents peuvent consister en des vitamines telles que l'ascorbate de sodium, le glutathion, ou d'autres produits (SOD) bien connus de l'homme du métier. En outre, l'installation peut également comprendre un dispositif permettant l'addition dans le produit sanguin de différents composés chimiques susceptibles d-'inactiver certains contaminants présents dans les produit sanguin à traiter. Ces composés peuvent être notamment des produits devenant actifs sous un rayonnement ultraviolet et susceptibles d'être combinés au procédé d'inactivation virale de manière à obtenir un effet synergique sur d'autres contaminants présents dans ledit produit sanguin. Cependant, il est important de noter que contrairement aux techniques utilisant des UV non pénétrants qui s'appliquent notamment aux produits sanguins présentés en fines couches, il est possible selon l'invention de traiter un produit sanguin sans recourir à l'application de fines couches et sans adjonction d'additifs toxiques d'inactivation virale.

La présente invention sera décrite de manière plus détaillée dans les exemples non limitatifs suivants en se référant aux figures annexées.

### Brève description des figures

- Les figures 1 et 2: représentent des exemples schématiques d'installation selon la présente invention.
- La figure 3: représente un détail schématique de l'installation selon la présente invention,
- La figure 4: représente le pourcentage de l'activité conservée du facteur VIII mesurée en milieu chromogène en fonction de doses d'irradiation croissantes de rayons ultraviolets reçues par le facteur VIII.
- La figure 5: représente le titrage du parvovirus MVMp inoculé dans une solution de facteur VIII traitée à des doses d'irradiation en rayons ultraviolets croissantes, les doses d'irradiation étant les doses reçues. Cette mesure est également représentée en donnant les valeurs logarithmiques.

### Description d'une forme d'exécution préférée de l'invention

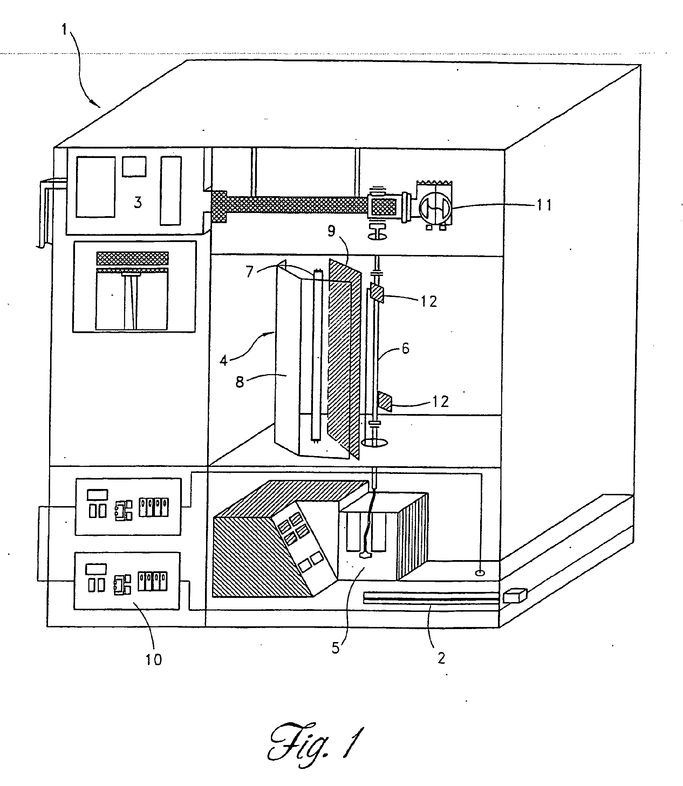

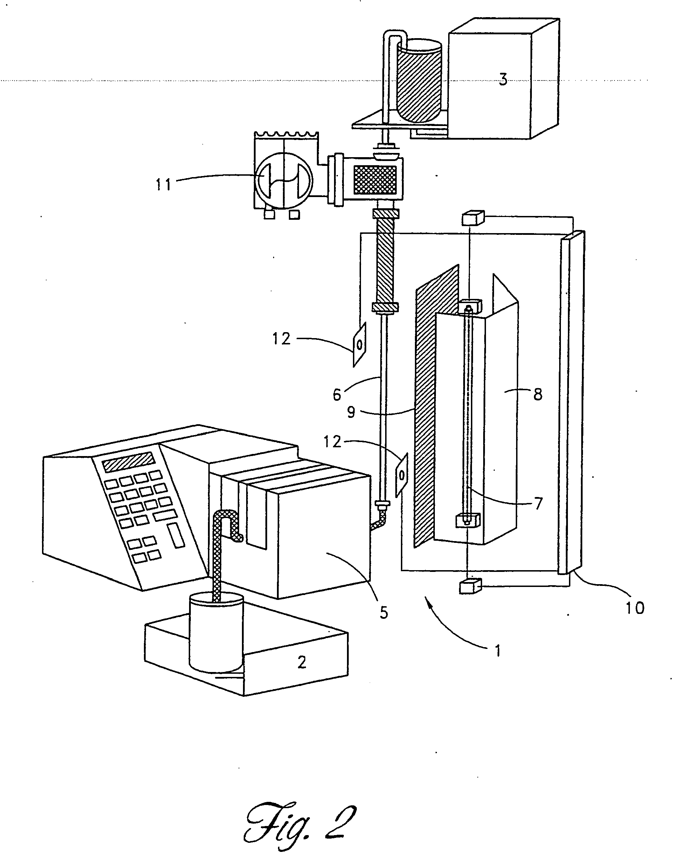

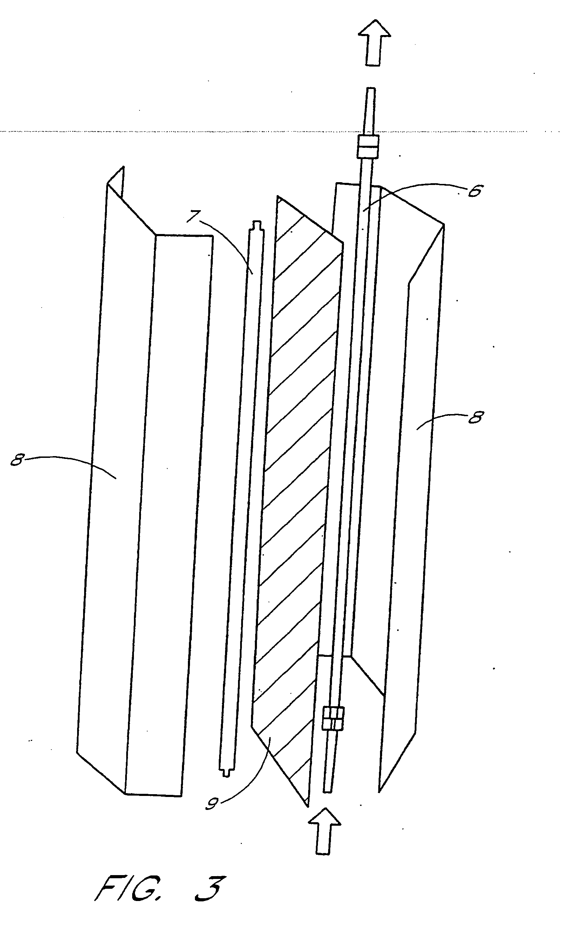

Dans les figures 1 à 3, on représente une installation pour la préparation d'un produit sanguin selon l'invention.

Cette installation 1 comprend des dispositifs (2,3) assurant notamment la précipitation, la centrifugation/décantation, filtration, concentration et dialyse de produits sanguins tels que le facteur VIII ou le fibrinogène, et adaptables par l'homme du métier en fonction d'un autre dérivé sanguin traité.

Cette installation comporte également le dispositif 4 assurant par un traitement physique, une inactivation virale dudit produit sanguin.

Le produit sanguin est amené par une pompe dans un tube en quartz 6 vers le dispositif 4.

Ce dispositif comprend une lampe UV 7, de préférence de type tube UV de désinfection, dont plus de 90% de l'émission s'effectue entre 230 et 270 nm, de préférence à une longueur d'ondes de l'ordre de 254 nm, cette lampe étant montée dans une enceinte réflectante 8, cylindrique ou non, qui renvoie le rayonnement vers le tube en quartz 6 disposé au niveau du foyer de l'enceinte réflectante 8.

Dans le dispositif, aucun contact n'est possible entre le produit circulant dans le tube en quartz 6 et la lampe UV 7.

Un système de turbulence tel qu'une chicane ou une injection d'azote permet de maintenir un flux homogène dans le tube en quartz 6.

L'installation comprend également une pompe 5 et un débitmètre 11 permettant de contrôler le débit du produit sanguin à traiter et de moduler le temps de passage du produit sanguin devant la lampe UV 7.

En outre, le dispositif peut comprendre un ou plusieurs filtres 9 disposés entre le tube en quartz 6 et la lampe UV 7. Le choix adéquat des filtres permet de moduler les doses d'irradiation reçues par le produit sanguin à traiter et les choix particuliers des longueurs d'onde émises. Il est également possible de moduler les doses d'irradiation reçues par le produit sanguin à traiter en adaptant le choix de la lampe UV utilisée (différentes puissances de lampe pouvant être utilisées), en sélectionnant les filtres utilisés et en adaptant le débit du produit sanguin passant devant la lampe. Ces modifications sont adaptables par l'homme du métier en fonction de la quantité et du type de produit sanguin traité. De plus, un système de contrôle 10 de la quantité d'ultraviolet C qui irradie le tube en quartz 6 (et donc la dose d'irradiation reçue par le produit sanguin) est placé du côté opposé par rapport à la lampe UV 7.

Ce système de contrôle comprend, ainsi que représenté dans les figures, un ou plusieurs capteur(s) 12 disposé(s) de manière avantageuse de chaque côté du tube en quartz 6 et éventuellement de chaque côté du filtre 9, de façon à permettre à l'homme du métier d'adapter le débit du flux du produit sanguin en fonction du type de produit sanguin à traiter et en fonction des doses d'irradiations émises par la lampe UV 7.

Le temps de résidence du produit sanguin peut être ajusté pour obtenir une dose constante d'irradiation. Le diamètre du tube peut être adapté au volume à traiter ainsi que la puissance ou la longueur de la lampe de désinfection. La température est contrôlée et enregistrée tant à l'intérieur du dispositif que dans le liquide (produit sanguin).

L'installation selon l'invention et le dispositif peuvent également comprendre des moyens de contrôle de la températures des produits sanguins, pouvant consister en des moyens de refroidissement tels qu'un dispositif réfrigérant ou un ventilateur.

Les différents matériaux utilisés dans le dispositif et l'installation selon l'invention sont avantageusement des produits essentiellement jetables tels que l'inox 316L, du téflon, ..., qui sont en accord avec les bonnes pratiques de production pharmaceutique (GMP) et qui peuvent être traités de manière sanitaire sur place.

Le dispositif d'inactivation virale par rayonnement ultraviolet C est avantageusement disposé en aval du procédé de traitement et de séparation général d'un produit sanguin, par exemple avant la filtration stérilisante ou après l'ultrafiltration du produit sanguin. La simplicité et le faible encombrement du dispositif mobile permet avantageusement son utilisation pour l'inactivation de n'importe quel type de produit sanguin sans modifier de manière exagérée une installation de préparation, de purification ou de séparation de produits sanguins.

Le dispositif et l'installation selon l'invention peuvent être construits d'un seul tenant ou en modules mobiles juxtaposés placés en série. Les doses d'irradiation reçues par le produit sanguin traité sont particulièrement faibles et varient entre 10 et 2.000 joules/m² et sont de préférence de l'ordre de 230 à 400 joules/m². De manière inattendue, ces doses d'irradiation suffisent pour obtenir l'inactivation virale recherchée.

La puissance de la lampe ultraviolet est avantageusement comprise de préférence entre 4 et 132 Watt, de préférence entre 8 et 60 Watt, de manière à respecter l'intégrité des produits traités. Il est à noter que par le procédé d'inactivation virale l'activité du produit sanguin (en particulier des facteurs de coagulation, du fibrinogène ou des immunoglobulines) est peu affectée (en moyenne moins de 5% de réduction d'activité).

La lampe UV utilisée dans l'installation selon l'invention est de préférence de type SPA®, en particulier celle produite par la société AQUAFIN VALENCIA (California U.S.A.)

Dans les exemples suivants, on donne différentes mesures d'inactivation virale obtenues sur des échantillons de produits sanguins infectés par des parvovirus et d'autres virus non enveloppés.

### Exemple

### 1. Matériel et Méthodes

En raison des problèmes que pose l'emploi de certains parvovirus humains et des problèmes de culture in vitro de ces parvovirus, en particulier le parvovirus B19, on utilise le parvovirus Murin MVMp de taille et de configuration très semblables comme modèle pour la mise au point de méthodes permettant l'inactivation du parvovirus B19. Le parvovirus Murin MVMp a été choisi, car ce type de parvovirus est moins sensible que le parvovirus B19 à une inactivation par rayonnement ultraviolet ou par modification de température.

Les tests sont comparés à l'inactivation d'un virus non enveloppé à RNA.

L'EMC (Encephalomyocarditis) est un membre de la famille des Picornaviridae, dont l'inactivation a été étudiée comme modèle de virus à RNA non enveloppé. Le virus EMC est un virus Murin utilisable comme modèle d'une contamination par le virus de l'hépatite A chez l'homme. 10⁶ pfu/ml pour EMC et 10¹² pfu/ml pour MVMp sont inoculés dans différents échantillons de produit sanguin (cryoprécipité, du facteur VIII ou des immunoglobulines).

### 2. Mesure de titrage du virus actif

L'index de réduction des virus a été déterminé selon les recommandations des Communautés Européennes (EEC Regulatory Document not for guidance, Biologicals 1991, 19, p.251) et exprimé en réduction logarithmique. Les mesures de titrage peuvent être réalisées selon les méthodes décrites par Tattersall P. (J. Virol. 10, pp. 586-590 (1972)) et par Russell S. J. Et al. (J. Virol. 66, pp. 2821-2828 (1992)).

Les lignées cellulaires choisies pour être infectées par les parvovirus sont la lignée cellulaire humaine NB324/k (décrite par Tattersall et al.) et la lignée L929 (clone 929 de la lignée A9 ATCC CCL 1.4).

Le titrage s'effectue par hybridation in situ des centres infectieux (centres réplicatifs) avec l'utilisation d'une sonde marquée radioactivement. La détection se faisant sur des filtres de nitrocellulose. La détermination du titre de virus peut être faite par plage de lyse ou par dilution limite (TCID50- méthode de Sperman-Kärber).

Les produits sanguins traités par le procédé sont un cryoprécipité de plasma, du facteur VIII, préalablement traité ou non par addition de solvant/détergent, du fibrinogène et des immunoglobulines.

### 3. Résultats

Le procédé (chaque étape) et l'installation de l'invention se conforment aux exigences des validations exigées par les autorités Européennes (CPMP/BWP/268/95 et CPMP/BWP/269/95 opérationnelles respectivement à partir du 14 août et du 13 septembre 1996 (incorporées ici par référence)). Conformément aux recommendations de ces autorités (§ 5.2.1 (1) CPMP/BWP/269/95), le procédé et l'installation de l'invention comportent au moins une étape opératoire de traitement efficace contre les virus non enveloppés en particulier le parvovirus B19 (§ 5.2.2 (iii)). L'inventicn satisfait en particulier les exigences d'inactivation requises à savoir réduction de 5 à 9 log (cf. Annexe I CPMP/BWP/269/95), c'est-à-dire qu'il est possible d'éliminer tous les virus inoculés. En effet, les Inventeurs n'ont observé après traitement aucune multiplication de virus au-dessus du seuil limite de détection.

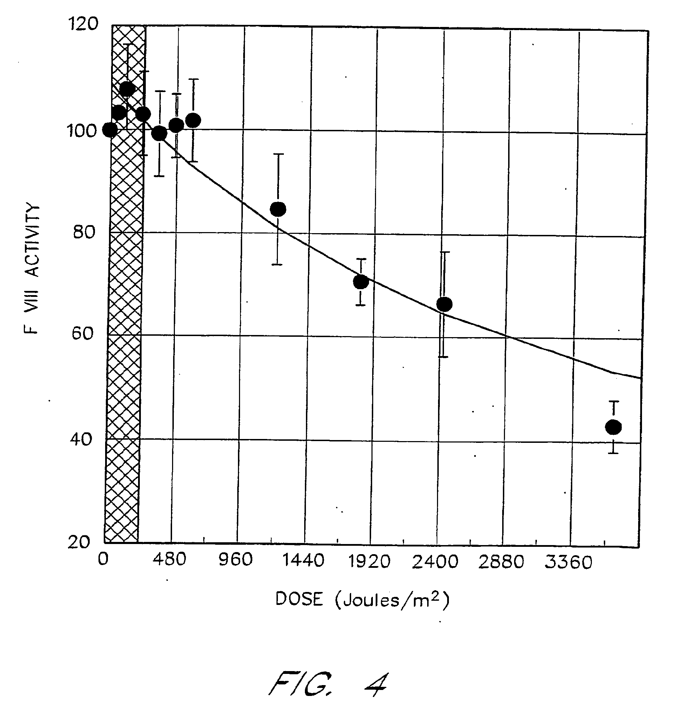

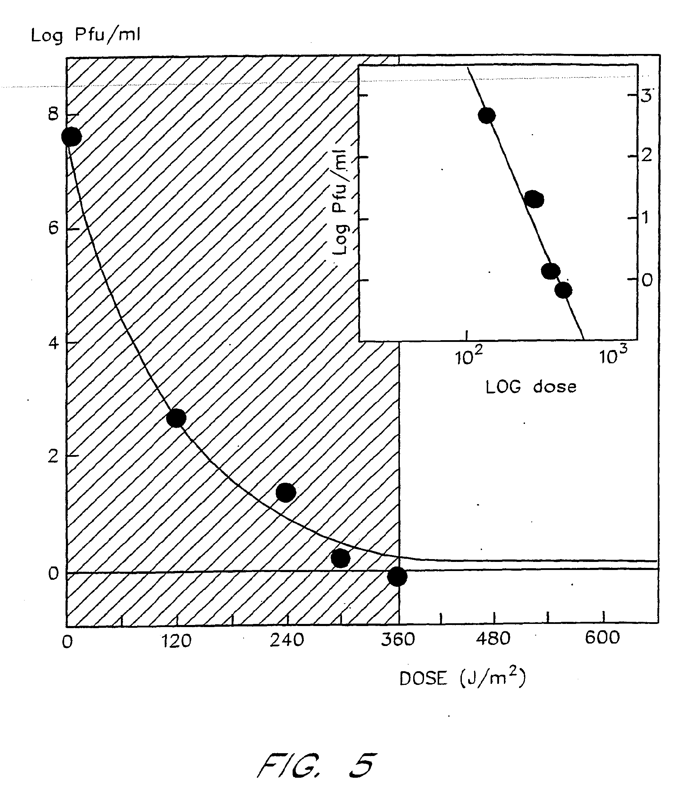

Comme il est indiqué dans la figure 4, des doses croissantes de rayonnement ultraviolet provoquent une inactivation des dérivés sanguins tels que le facteur VIII. Cependant, les Inventeurs ont observé de manière inattendue qu'il est possible d'obtenir une inactivation des parvovirus par irradiation au moyen de rayonnements ultraviolets C des virus inoculés dans des solutions comprenant du facteur VIII en limitant les doses d'irradiation sans affecter de manière importante l'activité des dérivés sanguins (voir figure 5).

Dans le tableau 1 ci-dessous, on observe une réduction logarithmique (log₁₀) des virus inoculés dans une composition comprenant des immunoglobulines. Ces valeurs de réduction logarithmique sont données pour des doses croissantes d'irradiation par rayonnement ultraviolet C reçues par lesdites immunoglobulines.

**Tableau 1**

| **Réduction logarithmique (log₁₀)** | | | | | | |
|---|---|---|---|---|---|---|
| **Type de virus** | **Dose (joules/m²)** | | | | | |
| | **60** | **120** | **180** | **240** | **600** | **1000** |
| **MVMp** | 3.08 | 4.73 | 5.60 | 6.33 | n.d. | n.d. |
| **EMC** | 1.53 | 3.04 | 3.84 | 4.49 | 5.07 | n.d. |

La concentration d'immunoglobulines dans la solution est de 2 mg/ml.

Des résultats similaires sont obtenus avec les autres produits sanguins traités.

## Revendications

1. Installation pour la préparation d'un produit sanguin comprenant un dispositif assurant une inactivation virale physique, le dispositif comportant :
- un émetteur de rayons ultraviolets de type C (UVC),
- un tube en quartz ou en un matériau polymérisé ne permettant pas l'absorption des rayonnements ultraviolets de type C, permettant, par un système de turbulence, de maintenir un flux homogène du produit sanguin circulant dans ce tube, dont le diamètre est adapté au volume à traiter afin que les produits sanguins ne soient pas en fines couches pour éviter les phénomènes de perturbations au niveau de la surface solide/liquide de ce tube,
- une enceinte réflectante renvoyant les rayonnements ultraviolets de type C vers le produit sanguin à traiter.

2. Installation selon la revendication 1, **caractérisée en ce que** le dispositif comporte un système de contrôle de la dose d'ultraviolets C qui irradie le produit sanguin à traiter.

3. Installation selon l'une quelconque des revendications 1 à 2, **caractérisée en ce que** le dispositif comporte un système de contrôle de la température du produit sanguin à traiter.

4. Installation selon la revendication 3, **caractérisée en ce que** la température est contrôlée et enregistrée tant a l'intérieur du dispositif que dans le produit sanguin à traiter.

5. Installation selon l'une quelconque des revendications 1 à 4, **caractérisée en ce que** les rayons ultraviolets de type C ont une longueur d'ondes comprise entre 250 et 270 nm, de préférence une longueur d'onde de 254 nm.

6. Installation selon l'une quelconque des revendications 1 à 5, **caractérisée en ce que** l'installation est construite d'un seul tenant ou en modules mobiles juxtaposés placés en série.

7. Utilisation d'un installation selon l'une quelconque des revendications 1 à 6, pour l'inactivation virale de virus non enveloppés présents dans un produit sanguin.

## Claims

1. Apparatus for the preparation of a blood product comprising a device allowing a physical inactivation of viruses, the device comprising :
- an emitter of type C ultraviolet radiation (UVC),
- a quartz tube or a tube made of a polymerized material which does not absorb type C ultraviolet radiation, allowing, through a system for turbulence, to maintain a homogeneous flow in the blood product circulating in said tube, of which the diameter is adapted to the volume to be treated so that the blood products are not in thin layers to avoid the perturbation phenomena at the solid/liquid surface of said tube
- a reflecting chamber sending the type C ultraviolet radiation towards the blood product to be treated.

2. Apparatus according to claim 1, **characterized in that** the device comprises a system for controlling the dose of the ultraviolet C radiation which irradiates the blood product to be treated.

3. Apparatus according to claim 1 or 2, **characterized in that** the device comprises a system for controlling the temperature of the blood product to be treated.

4. Apparatus according to claim 3, **characterized in that** the temperature is controlled and recorded inside the device as well as in the blood product to be treated.

5. Apparatus according to any of claims 1 to 4, **characterized in that** the type C ultraviolet radiation has a wavelength between 250 and 270 nm, preferably a wavelength of 254 nm.

6. Apparatus according to any of claims 1 to 5, **characterized in that** the apparatus is constructed in a single block or as juxtaposed portable modules placed in series.

7. Use of an apparatus according to any of claims 1 to 6, for the viral inactivation of non enveloped viruses present in a blood product.

## Patentansprüche

1. Einrichtung zur Aufbereitung eines Blutprodukts, die eine Vorrichtung aufweist, welche eine physikalische Inaktivierung eines Virus sicherstellt, wobei die Vorrichtung umfasst:
- einen Sender für ultraviolette Strahlen des Typs C (UVC),
- ein Rohr aus Quarz oder einem polymerisierten Material, das die Absorption ultravioletter Strahlen des Typs C nicht zulässt, und es durch ein auf Turbulenz basierendes System ermöglicht, einen homogenen Strom des Blutprodukts aufrechtzuerhalten, das in diesem Rohr zirkuliert, dessen Durchmesser an das zu behandelnde Volumen angepasst ist, damit die Blutprodukte nicht in feinen Schichten vorliegen, um die Phänomene von Störungen im Bereich der Feststoff/Flüssigkeitsoberfläche dieses Rohrs zu vermeiden,
- einen reflektierenden Schirm, der die ultraviolette Strahlung des Typs C in Richtung auf das zu behandelnde Blutprodukt lenkt.

2. Einrichtung nach Anspruch 1, **dadurch gekennzeichnet, dass** die Vorrichtung ein System zur Steuerung der Dosis der ultravioletten Strahlen C umfasst, die das zu behandelnde Blutprodukt bestrahlen.

3. Einrichtung nach einem der Ansprüche 1 bis 2, **dadurch gekennzeichnet, dass** die Vorrichtung ein System zur Steuerung der Temperatur des zu behandelnden Blutprodukts umfasst.

4. Einrichtung nach Anspruch 3, **dadurch gekennzeichnet, dass** die Temperatur im Inneren der Vorrichtung wie auch im zu behandelnden Blutprodukt gesteuert und aufgezeichnet wird.

5. Einrichtung nach einem der Ansprüche 1 bis 4, **dadurch gekennzeichnet, dass** die ultravioletten Strahlen des Typs C eine Wellenlänge zwischen 250 und 270 nm haben, vorzugsweise eine Wellenlänge von 254 nm.

6. Einrichtung nach einem der Ansprüche 1 bis 5, **dadurch gekennzeichnet, dass** die Einrichtung aus einem einzigen Teil aufgebaut ist, oder aus beweglichen Modulen, die in Reihe nebeneinander gesetzt werden.

7. Verwendung einer Einrichtung nach einem der Ansprüche 1 bis 6, zur viralen Inaktivierung von in einem Blutprodukt vorhandenen, nicht umhüllten Viren.
